# EUROPEAN PATENT APPLICATION

(11) **EP 4 527 451 A1**
(43) Date of publication of application: **26.03.2025**
(21) Application number: 23820041.4
(22) Date of filing: 01.06.2023
(51) Int. Cl.: A61N 7/02, G01B 7/00, A61N 7/00

(54) **ULTRASOUND GENERATION APPARATUS AND METHOD FOR DETERMINING AND CONTROLLING STATE THEREOF**

(30) Priority: 08.06.2022 KR 20220069586
(71) Applicant: Jeisys Medical Inc., Geumcheon-gu Seoul 08501 (KR)
(72) Inventor: RYU, Kwang Ho, Seoul 08501 (KR); KANG, Dong Hwan, Seoul 08501 (KR)
(74) Representative: dompatent von Kreisler Selting Werner - Partnerschaft von Patent- und Rechtsanwälten mbB
(86) International application number: PCT/KR2023/007488
(87) International publication number: WO 2023/239105

(57) **Abstract**

The present disclosure may include an ultrasonic generator; a transfer unit coupled to the ultrasonic generator and configured to move the ultrasonic generator in a preset pattern; a sensor configured to detect a movement of the ultrasonic generator; and a control unit configured to: determine a position of the ultrasonic generator based on detection result information by the sensor, and based on the position of the ultrasonic generator not changing while the transfer unit is being driven, determine that the ultrasonic generator is in one of a state of being separated from the transfer unit, a state of being detached, and a state of being stopped.

## Description

### [TECHNICAL FIELD]

The present disclosure relates to an ultrasonic generation device, and more particularly, to an ultrasonic generation device, and method for determining the state thereof and controlling the same.

### [BACKGROUND ART]

Ultrasound refers to waves with a frequency of 20 kHz or higher, and has the property of penetrating water, so it is widely used in the medical field, such as ultrasound diagnostic devices and ultrasound treatment devices.

The most representative application of ultrasound in the medical field is an ultrasound imaging device that utilizes the penetration and reflection properties of ultrasound. For example, there is a device that visualizes the time and intensity of reflection as ultrasound penetrates the human body and penetrates each organ, thereby obtaining a cross-sectional image of the human body.

In addition, there is a device that burns and removes specific subcutaneous tissues, such as tumors in the skin, or induces degeneration and regeneration of skin tissue by utilizing the heat generated by high intensity focused ultrasound (HIFU).

However, in the case of moving an ultrasonic generator, the conventional ultrasonic generation device has difficulty in accurately detecting the real-time position of the ultrasonic generator, making it difficult to efficiently irradiate ultrasound.

In addition, since the conventional ultrasonic generation device fastens a handpiece and a cartridge housing by magnetic force, there is a problem of performing repeated irradiation due to magnet detachment during high-speed ultrasonic irradiation.

Furthermore, the conventional ultrasonic generation device has a problem of thermal injury due to the inaccurate irradiation position of the ultrasonic generator.

In addition, the conventional ultrasonic generation device has a problem of causing an additional accident due to damage to driving parts because the driving device could be operated when the irradiation position of the ultrasonic generator is inaccurate.

In addition, the conventional ultrasonic generation device has a risk of burns when high heat and high ultrasonic energy are irradiated to a skin.

### [DETAILED DESCRIPTION OF THE INVENTION]

### [TECHINICAL PROBLEM]

A technical problem to solve by the present disclosure is to efficiently irradiate ultrasound by detecting a current position of an ultrasonic generator in real time.

In addition, a technical problem to solve by the present disclosure is to prevent repeated irradiation due to magnet detachment occurring during high-speed ultrasonic irradiation by reinforcing a physical fastening method in addition to a magnetic coupling method.

In addition, a technical problem to solve by the present disclosure is to stop an operation of a transducer to prevent thermal injury.

In addition, a technical problem to solve by the present disclosure is to prevent an additional accident while preventing damage to a driving component by stopping the operation of a driving device.

In addition, a technical problem to solve by the present disclosure is to stop an ultrasonic irradiation of a transducer in any one of an abnormally separated state, a detached state, and a stopped state, thereby preventing indiscriminate ultrasonic irradiation in advance, thereby preventing the occurrence of burns or skin troubles in advance.

In addition, a technical problem to solve by the present disclosure is to control high heat and high ultrasonic energy to prevent the occurrence of burn-related risks in advance.

The problems to be solved by the present disclosure are not limited to the problems mentioned above, and other problems not mentioned will be clearly understood by those skilled in the art from the description below.

### [TECHNICAL SOLUTION]

In an aspect of the present disclosure, an ultrasonic generation device may include an ultrasonic generator; a transfer unit coupled to the ultrasonic generator and configured to move the ultrasonic generator in a preset pattern; a sensor configured to detect a movement of the ultrasonic generator; and a control unit configured to: determine a position of the ultrasonic generator based on detection result information by the sensor, and based on the position of the ultrasonic generator not changing while the transfer unit is being driven, determine that the ultrasonic generator is in one of a state of being separated from the transfer unit, a state of being detached, and a state of being stopped.

Furthermore, two or more of the sensors may be installed at an equal interval along a moving path of the ultrasonic generator.

Furthermore, the control unit may be configured to determine the position of the ultrasonic generator based on magnetic force detection result information from the sensor.

Furthermore, the preset pattern may include at least one of a first pattern in which the ultrasonic generator moves from a first point to a second point, or a second pattern in which the ultrasonic generator moves from the second point to the first point, and the magnetic force detection result information may include multiple sensing points having a high intensity of the magnetic force among detected multiple magnetic forces, and multiple sensing points where the multiple magnetic forces intersect each other.

Furthermore, the control unit is further configured to: when the ultrasonic generator moves in one of the first pattern and the second pattern, stop an operation of the ultrasonic generator so that an ultrasound from the ultrasonic generator is not output based on the magnetic force detection result information being not based on the multiple sensing points with the high intensity of the magnetic force and the multiple sensing points where the multiple magnetic forces intersect each other. Preferably, when the second pattern is moved, in the case that the plurality of magnetic forces are not based on the multiple sensing points intersect each other, the operation of the ultrasonic generator may be further stopped so that the ultrasound of the ultrasonic generator are not output. This is because the second pattern is moved in a direction in which the transfer unit pulls the ultrasonic generator, so the ultrasonic generator may frequently be detached.

Furthermore, the device may further include a cartridge housing provided to accommodate the ultrasonic generator and is coupled to one side of a handpiece, wherein the handpiece may include an engaging projection formed on an inner surface and a main drive shaft having magnetism configured to couple with the cartridge housing, wherein the cartridge housing may include an insertion groove formed on an outer surface and a magnetic body provided to face the main drive shaft, and wherein the cartridge housing, when coupled with the handpiece, the insertion groove may be engaged by the engaging projection and is magnetically coupled with the main drive shaft having magnetism by the magnetic body.

In another aspect of the present disclosure, a method for determining a state and controlling an ultrasonic generation device may include moving an ultrasonic generator in a preset pattern; detecting a movement of the ultrasonic generator; and determining a position of the ultrasonic generator based on detection result information by a sensor, and based on the position of the ultrasonic generator not changing while the transfer unit is being driven, determining that the ultrasonic generator is in one of a state of being separated from the transfer unit, a state of being detached, and a state of being stopped.

Furthermore, the position of the ultrasonic generator may be determined based on magnetic force detection result information from the sensor.

Furthermore, the preset pattern may include at least one of a first pattern in which the ultrasonic generator moves from a first point to a second point, or a second pattern in which the ultrasonic generator moves from the second point to the first point, and the magnetic force detection result information may include multiple sensing points having a high intensity of the magnetic force among detected multiple magnetic forces, and multiple sensing points where the multiple magnetic forces intersect each other.

Furthermore, when the ultrasonic generator moves in one of the first pattern and the second pattern, the method may further include stopping an operation of the ultrasonic generator so that an ultrasound from the ultrasonic generator is not output based on the magnetic force detection result information being not based on the multiple sensing points with the high intensity of the magnetic force and the multiple sensing points where the multiple magnetic forces intersect each other.

### [ADVANTAGEOUS EFFECTS OF THE INVENTION]

According to the present disclosure, there is an effect of efficiently irradiating ultrasound by detecting the current position of the ultrasonic generator in real time.

In addition, according to the present disclosure, there is an effect of preventing repeated irradiation due to magnet detachment that occurs during high-speed ultrasonic irradiation by reinforcing a physical fastening method in addition to a magnetic coupling method.

In addition, according to present disclosure, there is an effect of preventing thermal injury by stopping the operation of the transducer.

In addition, according to the present disclosure, there is an effect of preventing an additional accident while preventing damage to the driving part by stopping the operation of the driving device.

In addition, according to the present disclosure, there is an effect of preventing indiscriminate ultrasonic irradiation in advance by stopping the ultrasonic irradiation of the transducer in any one of an abnormally separated state, a detached state, and a stopped state, thereby preventing the occurrence of burns or skin troubles in advance.

In addition, according to present disclosure, there is an effect that can prevent the occurrence of a risk due to burns in advance by controlling high heat and high ultrasonic energy.

Technical effects of the inventive concept are not limited to the technical effects mentioned above, and other technical effects not mentioned will be clearly understood by those skilled in the art from the following description.

### [DESCRIPTION OF THE DRAWINGS]

FIG. 1 is a perspective view illustrating an ultrasonic generation device according to the present disclosure.
FIG. 2 is an exploded perspective view illustrating an ultrasonic generation cartridge.
FIG. 3 is a cross-sectional view illustrating the ultrasonic generation cartridge of FIG. 1.
FIG. 4 is a cross-sectional view illustrating a process of combining the cartridge housing and the handpiece of FIG. 1.
FIG. 5 is a diagram illustrating a configuration of the ultrasonic generation device according to the present disclosure.
FIG. 6 is a diagram illustrating an example of the process of the ultrasonic generator of FIG. 5 moving.
FIG. 7 is a diagram illustrating an example of a pattern in which the ultrasonic generator of FIG. 6 moves.
FIG. 8 is a flowchart illustrating a method for controlling an ultrasonic generation device and determining a state thereof according to the present disclosure.
FIGS. 9 to 11 are diagrams illustrating an example of a process of detecting the magnetic force of the magnet generated when the transducer moves by the two or more sensors installed in FIG. 6.
FIG. 12 is a flowchart illustrating an example of a method of irradiating ultrasonic energy by determining whether the ultrasonic generator and the driving device are driven based on the state determination of the ultrasonic generator of FIG. 8.
FIG. 13 is a diagram illustrating an example of a process of outputting magnetic detection result information through the processor when the ultrasonic generator of FIG. 7 moves forward in the first pattern.
FIG. 14 illustrates a cushioning package material using a nonwoven fabric according to a sixth embodiment of the present disclosure.
FIG. 15 is a diagram illustrating an example of a process for outputting magnetic detection result information through the processor when the ultrasonic generator of FIG. 7 moves back and forth in the first pattern and the second pattern.

### [BEST MODE]

In the drawings, the same reference numeral refers to the same element. This disclosure does not describe all elements of embodiments, and general contents in the technical field to which the present disclosure belongs or repeated contents of the embodiments will be omitted. The terms, such as "unit, module, member, and block" may be embodied as hardware or software, and a plurality of "units, modules, members, and blocks" may be implemented as one element, or a unit, a module, a member, or a block may include a plurality of elements.

Throughout this specification, when a part is referred to as being "connected" to another part, this includes "direct connection" and "indirect connection", and the indirect connection may include connection via a wireless communication network.

Furthermore, when a certain part "includes" a certain element, other elements are not excluded unless explicitly described otherwise, and other elements may in fact be included.

In the entire specification of the present disclosure, when any member is located "on" another member, this includes a case in which still another member is present between both members as well as a case in which one member is in contact with another member.

The terms "first," "second," and the like are just to distinguish an element from any other element, and elements are not limited by the terms.

The singular form of the elements may be understood into the plural form unless otherwise specifically stated in the context.

Identification codes in each operation are used not for describing the order of the operations but for the convenience of description, and the operations may be implemented differently from the order described unless there is a specific order explicitly described in the context.

Hereinafter, operation principles and embodiments of the present disclosure will be described with reference to the accompanying drawings.

First, the High Intensity Focused Ultrasound (HIFU) technology is the latest thermal ablation treatment that burns specific subcutaneous tissues such as tumors in the skin by using the heat generated when high-intensity ultrasound is focused on one point in the skin. This is similar to the principle of focusing warm sunlight with a magnifying glass to light a fire. Since ultrasound easily passes through body tissues, HIFU treatment is performed in a completely non-invasive manner without a knife or even a needle. In other words, by simply pressing the patient's skin on the ultrasound generation surface, specific subcutaneous tissues such as tumors are burned and treated. In addition, HIFU treatment is currently being used to treat uterine fibroids, bone metastases, prostate cancer, breast cancer, pancreatic cancer, liver cancer, and kidney cancer.

This high-intensity focused ultrasound technology may be implemented through an ultrasound generation cartridge. The ultrasound generation cartridge may irradiate ultrasound energy to the surface of the patient's skin.

The control unit of the ultrasonic generation device according to the present disclosure in this specification includes various devices that may perform computational processing and provide a result to a user. For example, the control unit of the ultrasonic generation device according to the present disclosure may include a computer, a server device, and a portable terminal, or may be in the form of one of them.

Here, the computer may include, for example, a notebook, a desktop, a laptop, a tablet PC, a slate PC, and the like mounted with a web browser.

The server device is a server that communicates with an external device to process information, and may include an application server, a computing server, a database server, a file server, a mail server, a proxy server, and a web server.

A portable terminal is a wireless communication device that ensures portability and mobility, and may include all kinds of handheld-based wireless communication devices such as PCS (Personal Communication System), GSM (Global System for Mobile communications), PDC (Personal Digital Cellular), PHS (Personal Handyphone System), PDA (Personal Digital Assistant), IMT (International Mobile Telecommunication)-2000, CDMA (Code Division Multiple Access)-2000, W-CDMA (W-Code Division Multiple Access), WiBro (Wireless Broadband Internet) terminals, smart phones, and the like, and wearable devices such as watches, rings, bracelets, anklets, necklaces, glasses, contact lenses, or head-mounted devices (HMDs).

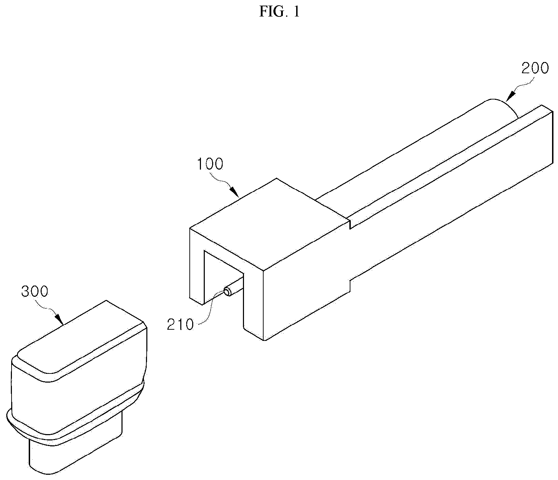

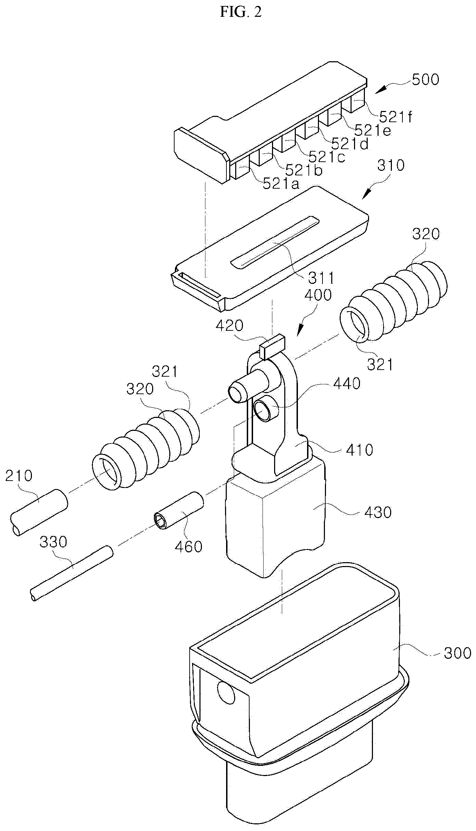

FIG. 1 is a perspective view illustrating an ultrasonic generation device according to the present disclosure. FIG. 2 is an exploded perspective view illustrating an ultrasonic generation cartridge.

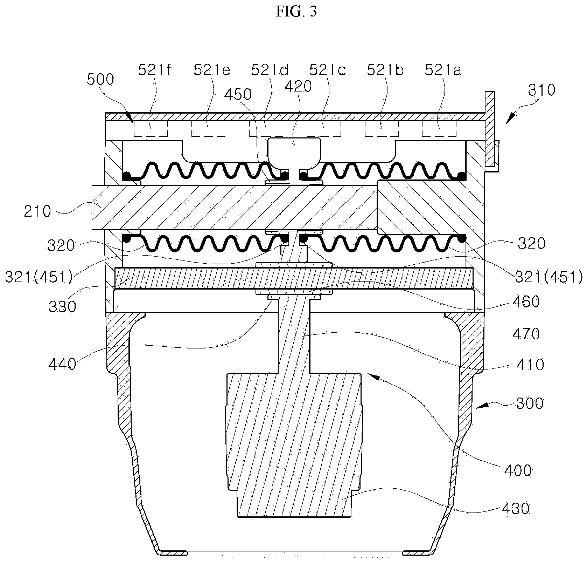

FIG. 3 is a cross-sectional view illustrating the ultrasonic generation cartridge of FIG. 1.

As shown in FIGS. 1 to 3, the ultrasonic generation device may include a handpiece 100, a driving device 200, and a cartridge housing 300.

The handpiece 100 is a basic body that may be used as a handle for the user to hold, and the cartridge housing 300 that accommodates an ultrasonic generator 400 may be detachably coupled to one side of the handpiece 100.

A user may hold the handpiece 100 and move the handpiece 100 so that the cartridge housing 300 is in close contact with a skin surface, and then irradiate high-intensity focused ultrasound generated from the ultrasonic generator 400 to a specific depth of the skin.

Inside the handpiece 100, a cable connected to an RF board for applying RF current to the driving device 200 and the ultrasonic generator 400 may be provided. The RF board may be accommodated in the cartridge housing 300, and may intermittently or continuously apply RF current to the driving device 200 and the ultrasonic generator 400.

The driving device 200 serves to provide power for moving the ultrasonic generator 400. The driving device 200 may move the ultrasonic generator 400 through a main driving shaft 210 that is connected through a mounter 450 of the ultrasonic generator 400. For example, the driving device 200 may be an actuator or motor that reciprocally moves the main driving shaft 210. In addition, the direction in which the driving device 200 moves the main driving shaft 210 may be the longitudinal direction of the handpiece 100 or the longitudinal direction of the cartridge housing 300. In addition, the driving device 200 may be installed in the handpiece 100 or the cartridge housing 300.

The cartridge housing 300 is a kind of case that accommodates the ultrasonic generator 400 and may be detachably coupled to one side of the handpiece 100. The medium of the high-intensity focused ultrasound generated from the ultrasonic generator 400 may be accommodated inside the cartridge housing 300. For example, the medium may be distilled water, degassed liquid, silicone, and the like, but the present disclosure is not particularly limited thereto.

The ultrasonic generator 400 is provided to be reciprocally movable in the cartridge housing 300 and generates high-intensity focused ultrasound. The ultrasonic generator 400 may include a frame 410, a magnet 420, a transducer 430, a slider 440, a mounter 450, a bushing 460, and the like.

Here, the frame 410, the magnet 420, the transducer 430, the slider 440, the mounter 450, and the bushing 460 of the ultrasonic generator 400 may be integrally connected and move together.

In addition, the movement of the ultrasonic generator 400 may be performed by the driving device 200 that moves the main driving shaft 210 coupled to the mounter 450 of the ultrasonic generator 400.

The frame 410 may be a basic body and may be integrally connected with the magnet 420, the slider 440, and the mounter 450. The magnet 420 may be connected to an upper side of the frame 410, and a transducer 430 may be detachably coupled to a lower side of the frame 410. In addition, the mounter 450 and the slider 440 may be coupled through and through between the magnet 420 and the transducer 430 in the frame 410, and the mounter 450 may be placed between the magnet 420 and the slider 440 in the frame 410. In addition, the central axis of the magnet 420 and the central axis of the transducer 430 may be arranged to be aligned with the central axis of the frame 410.

A detection unit 500 may be provided to face the magnet 420 and may include two or more sensors 521a to 521f installed at an equal interval along the movement path of the ultrasonic generator 400. The two or more sensors 521a to 521f may each detect the magnetic force of the magnet 420 generated when the ultrasonic generator 400 moves. For example, the two or more sensors 521a to 521f may be magnetic Hall sensors. The magnetic Hall sensors may linearly detect a current position of the transducer 430 based on the strength of the magnetic force measured according to the positional relationship with the magnet 420. These magnetic Hall sensors may detect all values of the change in the intensity of the magnetic force in real time, so that they may detect even the part where the intensity of the magnetic force between the sensors overlaps, so that at least one of a separated state, a detached state, or stopped state of the transducer 430 may be detected during the ultrasonic irradiation.

A guide plate 310 covers the upper part of the cartridge housing 300, and a slot 311 for guiding the movement of the magnet 420 may be formed in the center of the guide plate 310. Here, the slot 311 may be formed along the movement direction of the magnet 420.

The transducer 430 may receive RF current from the RF board of the handpiece 100 to generate high-intensity focused ultrasound.

The mounter 450 is provided between the magnet 420 and the transducer 430, and may be detachably connected to the end of the main drive shaft 210 described above, or may be integrally connected. Accordingly, when the drive device 200 moves the main drive shaft 210, the mounter 450 may also move together.

For example, both sides of the mounter 450 may be supported by a pair of bellows 320. The pair of bellows 320 may be arranged with the mounter 450 in between. For example, one end of the bellows 320 may be detachably connected to one side in the direction of movement of the mounter 450, and the other end of the bellows 320 may be in contact with the inner surface of the cartridge housing 300.

Accordingly, when the mounter 450 moves, one of the pair of bellows 320 is compressed and the other is expanded, so that the pair of bellows 320 may support the mounter 450.

Meanwhile, a protruding insertion projection 321 may be formed on one of the bellows 320 and the mounter 450, and a recessed groove 451 in which the insertion projection 321 is detachably coupled may be formed on the other of the bellows 320 and the mounter 450. For example, referring to FIG. 3, since the insertion projection 321 is formed at one end of the bellows 320 and the recessed groove 451 is formed on the outer surface of the mounter 450, the insertion projection 321 of the bellows 320 may be detachably coupled to the recessed groove 451 of the mounter 450. More specifically, the insertion projection 321 of the bellows 320 and the recessed groove 451 of the mounter 450 may be formed in a ring shape.

The slider 440 may be provided between the magnet 420 and the transducer 430. This slider 440 may slide along a guide shaft 330 that is arranged parallel to the moving direction of the magnet 420 in the cartridge housing 300. Here, both ends of the guide shaft 330 are fixed to both sides of the cartridge housing 300, and the guide shaft 330 may penetrate the slider 440 to guide the sliding of the slider 440.

The bushing 460 is provided on an inner surface of the slider 440, and may play a role in reducing wear of the slider 440 when the slider 440 slides along the guide shaft 330. The bushing 460 may have a shape that protrudes to both sides of the slider 440.

The ultrasonic generation device according to the present disclosure may be provided to prevent detachment of the transducer 430 when the cartridge housing 300 is coupled to the handpiece 100, and to further enhance the fastening force together with the coupling by magnetic force.

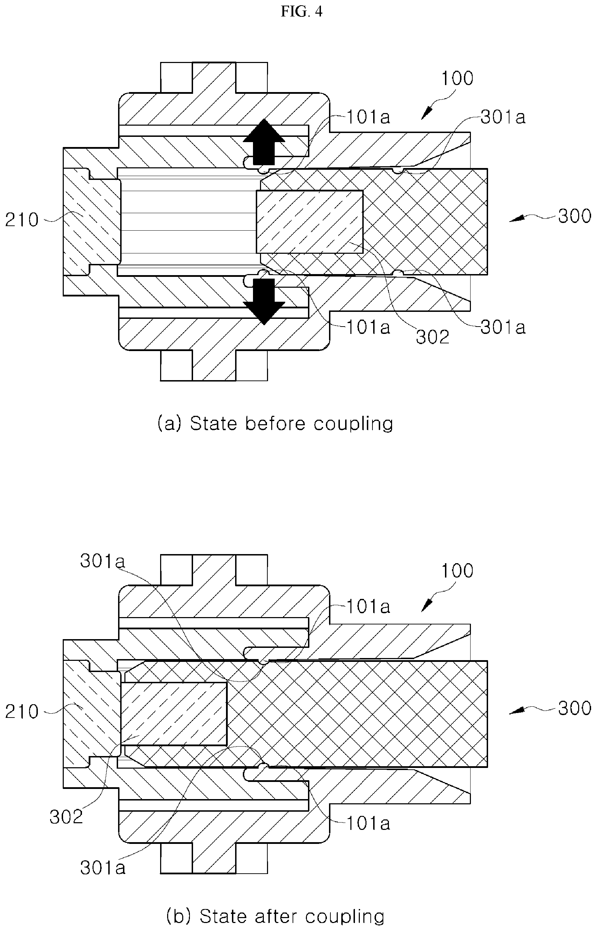

FIG. 4 is a cross-sectional view illustrating a process of combining the cartridge housing and the handpiece of FIG. 1.

As shown in FIG. 4, the cartridge housing 300 may be coupled with one side of the handpiece 100. The handpiece 100 may include an engaging projection 101a formed on the inner surface and the main driving shaft 210 having magnetism for coupling with the cartridge housing 300. For example, the engaging projection 101a may be a cantilever-shaped hook.

The cartridge housing 300 may include an insertion groove 301a formed on the outer surface and a magnetic body 302 arranged to face the main driving shaft 210. When the cartridge housing 300 is coupled with the handpiece 100, the insertion groove 301a may be engaged by the engaging projection 101a and may be magnetically combined with the main driving shaft 210 having magnetism by the magnetic body 302. For example, the magnetic body 302 may be provided at a tip of the cartridge housing 300 and may be magnetically coupled to the end of the main drive shaft 210 having magnetism.

The ultrasonic generation device according to the present disclosure may be provided to determine at least one of the real-time position of the ultrasonic generator 400 and whether the ultrasonic generator 400 is separated from the transfer unit, detached, or stopped based on the magnetic force detection result information by two or more sensors 521a to 521f when the ultrasonic generator 400 moves.

Hereinafter, the process for determining the state of the ultrasonic generation device and controlling the operation of the ultrasonic generation device will be examined in detail.

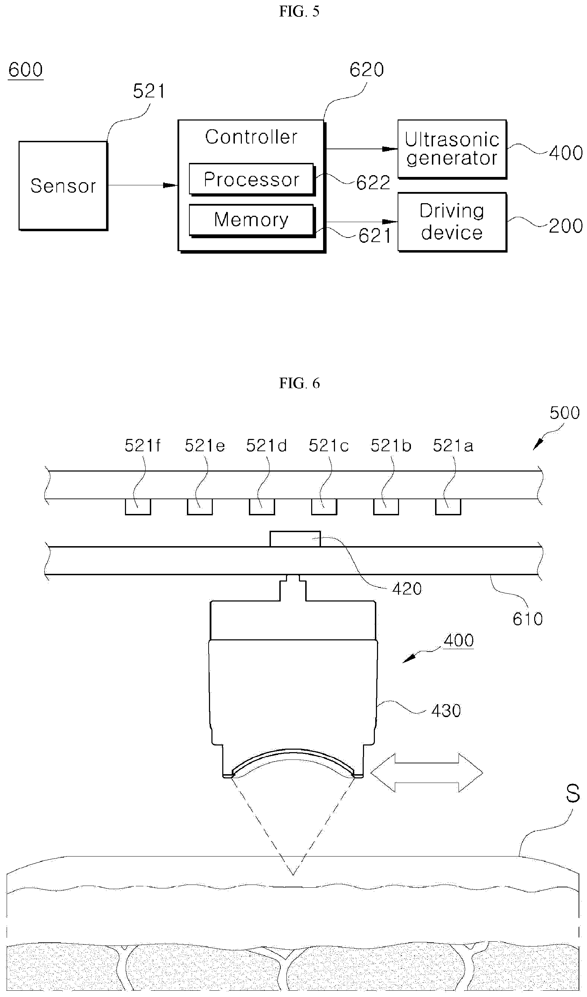

FIG. 5 is a diagram illustrating a configuration of the ultrasonic generation device according to the present disclosure. FIG. 6 is a diagram illustrating an example of the process of the ultrasonic generator of FIG. 5 moving.

As shown in FIGS. 5 and 6, the ultrasonic generation device 600 may include the driving device 200, a transfer unit 610, the ultrasonic generator 400, a sensor 521, and a control unit 620.

The transfer unit 610 may be provided to support the ultrasonic generator 400 and move the ultrasonic generator 400 to left or right. Here, the transfer unit 610 will be described as the main driving shaft for the convenience of description. The transfer unit 610 may move the ultrasonic generator 400 to the left or right according to the driving of the driving device 200. The ultrasonic generator 400 having the transducer 430 may be moved to the left or right along the horizontal direction while maintaining the ultrasonic focus depth by the movement of the transfer unit 610. The transfer unit 610 is coupled with the ultrasonic generator 400 and may move the ultrasonic generator 400 in a preset pattern.

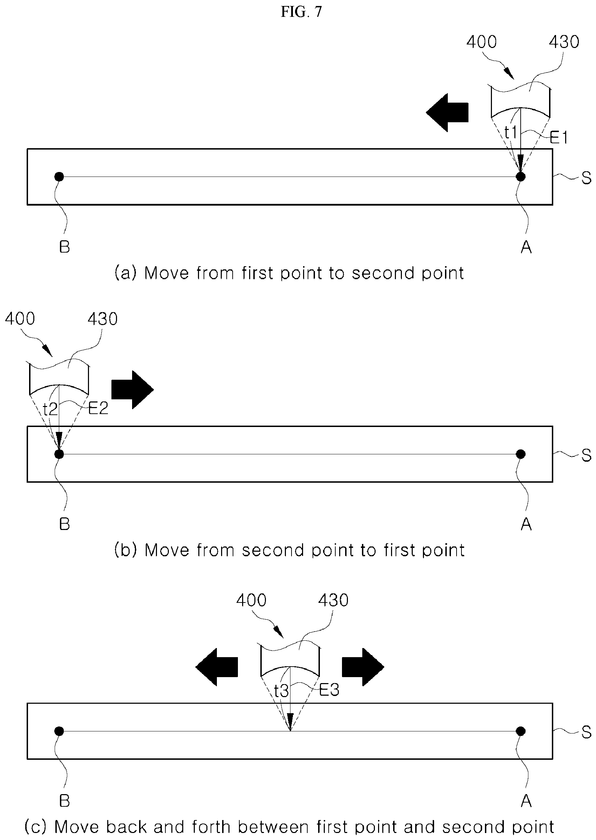

FIG. 7 is a diagram illustrating an example of a pattern in which the ultrasonic generator of FIG. 6 moves.

Referring to FIG. 7, the transfer unit 610 may move the ultrasonic generator 400 in a preset pattern. The preset pattern may include a first pattern in which the ultrasonic generator 400 moves from a first point A to a second point B, as shown in (a) of FIG. 7. The preset pattern may include a second pattern in which the ultrasonic generator 400 moves from the second point B to the first point A, as shown in (b) of FIG. 7. The preset pattern may include the first pattern and the second pattern in which the ultrasonic generator 400 moves back and forth between the first point A and the second point B, as shown in (c) of FIG. 7. Information about the first pattern and information about the second pattern may be stored in a memory 621.

The control unit 620 may be implemented as the memory 621 that stores data on an algorithm for controlling the operation of components within the device or a program that reproduces the algorithm, and at least one processor 622 that performs the operation described above using the data stored in the memory 621. Here, the memory 621 and the processor 622 may each be implemented as separate chips. In addition, the memory 621 and the processor 622 may be implemented as a single chip.

The memory 621 may store data supporting various functions of the device, a program for the operation of the control unit, may store input/output data, and may store a plurality of application programs or applications run on the device, data for the operation of the device, and commands. At least some of these application programs may be downloaded from an external server via wireless communication.

The memory 621 may include at least one type of storage medium among a flash memory type, a hard disk type, an SSD type (Solid State Disk type), an SDD type (Silicon Disk Drive type), a multimedia card micro type, a card type memory (for example, an SD or XD memory, etc.), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. In addition, the memory 120 may be a database that is separate from the device but connected by wire or wirelessly.

As illustrated in FIG. 6, the detection unit 500 may detect magnetic force generated when the ultrasonic generator 400 moves. The detection unit 500 may include at least one sensor 521 installed along the movement path of the ultrasonic generator 400. For example, the detection unit 500 may include two or more sensors 521a to 521f installed at an equal interval along the movement path of the ultrasonic generator 400. Each of the two or more sensors 521a to 521f may detect the magnetic force of the magnet 420 generated when the transducer 430 of the ultrasonic generator 400 moves.

The processor 622 may determine the position of the ultrasonic generator based on the magnetic force detection result information by the sensor 521.

The processor 622 may determine at least one of the current position of the transducer 430, the movement stop phenomenon of the transducer 430, and whether the transducer 430 is separated from the transfer unit 610 based on the magnetic force detection result information.

The processor 622 may determine that the ultrasonic generator 400 is in one of the following states: separated, detached, or stopped, in the case that the position of the ultrasonic generator 400 does not change while the driving device 200 driving the ultrasonic generator 400 is being driven.

For example, the processor 622 may determine the current position of the transducer 430 based on the magnetic detection result information by the two or more sensors 521a to 521f. In the case that the position of the transducer 430 changes normally while the transfer unit 610 is being driven, the processor 622 may drive the transducer 430 to irradiate ultrasound to the skin S on the movement path of the transfer unit 610 through the transducer 430.

On the other hand, in the case that there is no change in the position of the transducer 430 while the transfer unit 610 is being driven, the processor 622 may determine that the transducer 430 has stopped moving or that the transducer 430 has been abnormally separated (detached) from the transfer unit 610. In the case that there is no change in the position of the transducer 430, the processor 622 may stop the driving of the transducer 430 to prevent thermal injury. In addition, in the case that there is no change in the position of the transducer 430, the processor 622 may stop the driving of the driving device 200 while stopping the driving of the transducer 430, thereby preventing damage to driving components and preventing an additional accident. At this time, the processor 622 may stop the driving of the transducer 430 so that ultrasound is not irradiated while the transducer 430 is separated.

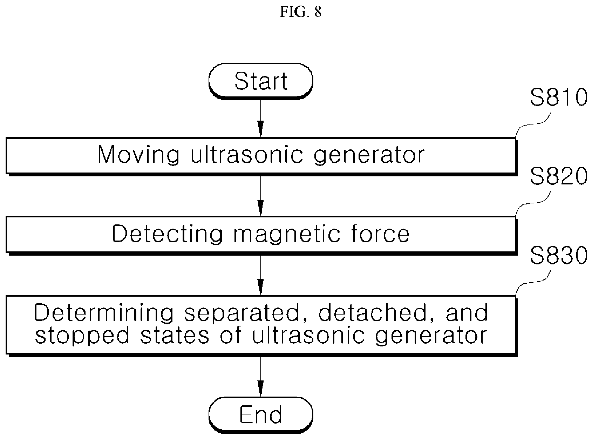

FIG. 8 is a flowchart illustrating a method for controlling an ultrasonic generation device and determining a state thereof according to the present disclosure.

Referring to FIG. 8, the method for controlling an ultrasonic generation device and determining a state thereof may include a step of moving the ultrasonic generator (step S810), a step of detecting magnetic force (step S820), and a step of determining the state of the ultrasonic generator (step S830).

The step of moving the ultrasonic generator may move the ultrasonic generator 400 in a preset pattern through the transfer unit 610 (step S810). The preset pattern may include the first pattern in which the ultrasonic generator 400 moves from the first point A to the second point B, as shown in (a) of FIG. 7. The preset pattern may include the second pattern in which the ultrasonic generator 400 moves from the second point B to the first point A, as shown in (b) of FIG. 7. The preset pattern may include the first pattern and the second pattern in which the ultrasonic generator 400 moves back and forth between the first point A and the second point B, as shown in (c) of FIG. 7. Information about the first pattern and information about the second pattern may be stored in the memory 621.

The step of detecting magnetic force may detect the magnetic force generated when the ultrasonic generator 400 moves through the detection unit 500 (step S820). The detection unit 500 may include at least one sensor 521 installed along the movement path of the ultrasonic generator 400. For example, the step of detecting magnetic force may detect the magnetic force of the magnet 420 generated when the ultrasonic generator 400 moves by the two or more sensors 521a to 521f installed at an equal interval along the movement path of the ultrasonic generator 400. For example, the two or more sensors 521a to 521f may be magnetic Hall sensors.

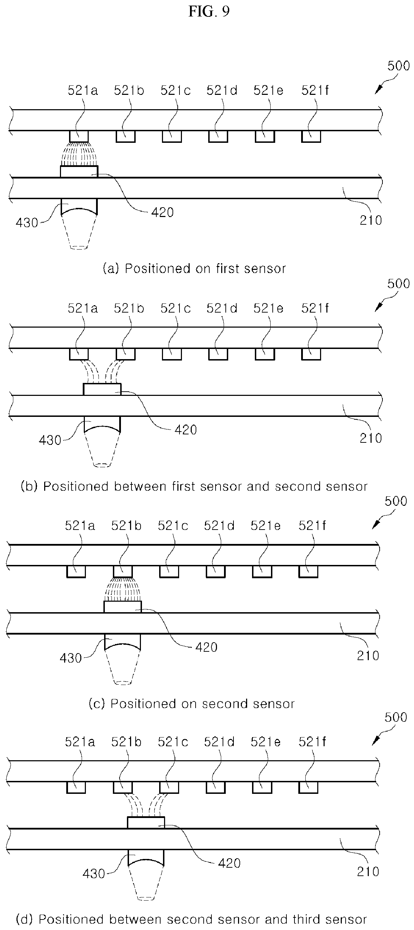

FIGS. 9 to 11 are diagrams illustrating an example of a process of detecting the magnetic force of the magnet generated when the transducer moves by the two or more sensors installed in FIG. 6.

First, the transducer 430 according to the present disclosure may move in the first pattern from the first point A to the second point B as shown in (a) of FIG. 7, may move in the second pattern from the second point B to the first point A as shown in (b) of FIG. 7, and may move in the first pattern and the second pattern reciprocating between the first point A and the second point B as shown in (c) of FIG. 7.

Hereinafter, for the convenience of description, the transducer 430 is described by illustrating the first pattern in which the transducer 430 moves from the first point A to the second point B.

Referring to (a) and (b) of FIG. 9, the step of detecting magnetic force may detect the magnetic force of the magnet 420 generated when the transducer 430 moves through the first sensor 521a when the transducer 430 changes to the position of the first sensor 521a. The step of detecting magnetic force may detect the magnetic force of the magnet 420 generated when the transducer 430 moves through the first sensor 521a and the second sensor 521b when the transducer 430 is changed to the position between the first sensor 521a and the second sensor 521b.

Referring to (c) and (d) of FIG. 9, the step of detecting magnetic force may detect the magnetic force of the magnet 420 generated when the transducer 430 moves through the second sensor 521b when the transducer 430 is changed to the position between the first sensor 521a and the second sensor 521b. The step of detecting magnetic force may detect the magnetic force of the magnet 420 generated when the transducer 430 moves through the second sensor 521b and the third sensor 521c when the transducer 430 is changed to the position between the second sensor 521b and the third sensor 521c.

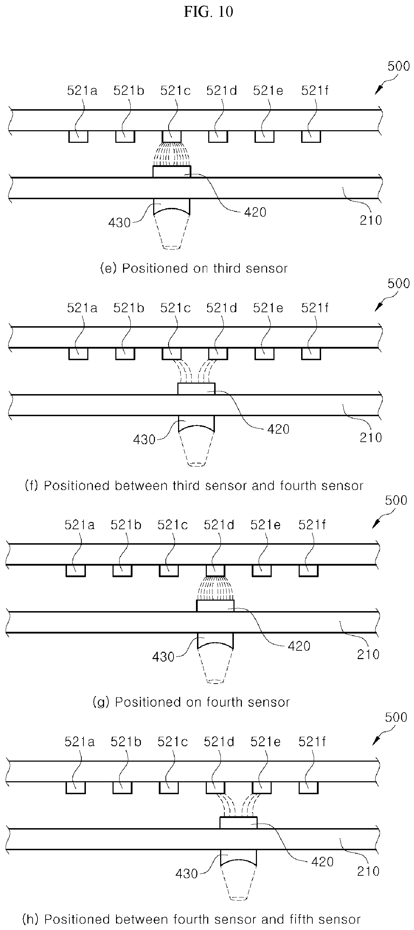

Referring to (e) and (f) of FIG. 10, the step of detecting magnetic force may detect the magnetic force of the magnet 420 generated when the transducer 430 moves through the third sensor 521c when the transducer 430 is changed to the position between the second sensor 521b and the third sensor 521c. The magnetic force detection step may detect the magnetic force of the magnet 420 generated when the transducer 430 moves through the third sensor 521c and the fourth sensor 521d when the transducer 430 is changed to the position between the third sensor 521c and the fourth sensor 521d.

Referring to (g) and (h) of FIG. 10, the step of detecting magnetic force may detect the magnetic force of the magnet 420 generated when the transducer 430 moves through the fourth sensor 521d when the transducer 430 is changed to the position between the third sensor 521d. The step of detecting magnetic force may detect the magnetic force of the magnet 420 generated when the transducer 430 moves through the fourth sensor 521d and the fifth sensor 521e when the transducer 430 is changed to the position between the fourth sensor 521d and the fifth sensor 521e.

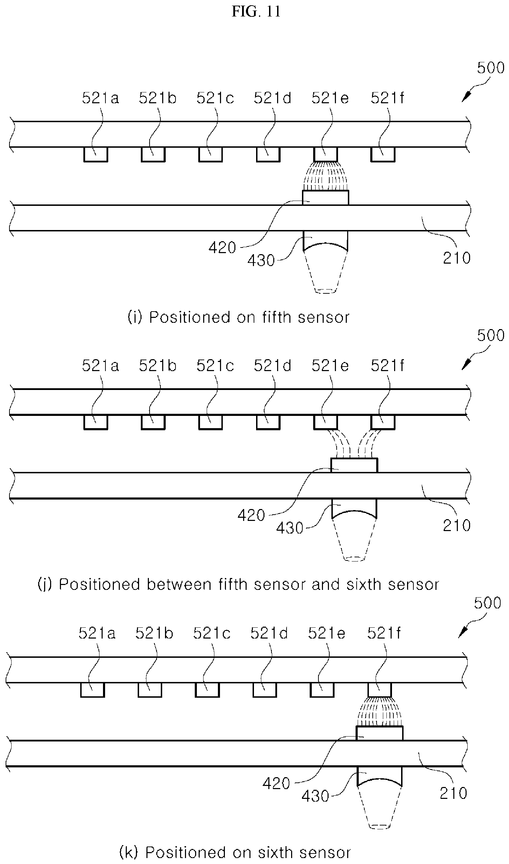

Referring to (i), (j), and (k) of FIG. 11, the step of detecting magnetic force may detect the magnetic force of the magnet 420 generated when the transducer 430 moves through the fifth sensor 521e when the transducer 430 is changed to the position between the fourth sensor 521d and the fifth sensor 521e. The step of detecting magnetic force may detect the magnetic force of the magnet 420 generated when the transducer 430 moves through the fifth sensor 521e and the sixth sensor 521f when the transducer 430 is changed to the position between the fifth sensor 521e and the sixth sensor 521f. The step of detecting magnetic force may detect the magnetic force of the magnet 420 generated when the transducer 430 moves through the sixth sensor 521f when the transducer 430 is changed to the position between the fifth sensor 521e and the sixth sensor 521f.

As illustrated in FIGS. 9 to 11, the two or more sensors 521a to 521f may linearly detect the current position of the transducer 430 based on the strength of the magnetic force measured according to the positional relationship with the magnet 420. These two or more sensors 521a to 521f may detect all values of the change in the intensity of the magnetic force in real time, so that even the part where the intensity of the magnetic force between the sensors overlaps may be detected, so that at least one of the separated state, detached state, or stopped state of the transducer 430 may be detected during the ultrasonic irradiation. Here, for the convenience of description, the two or more sensors 521f are illustrated as being provided in six units, but may be provided in less than six or more than six units depending on the ultrasonic irradiation conditions and internal space, and the like.

The step of determining the state of the ultrasonic generator may determine the position of the ultrasonic generator 400 based on the magnetic force detection result information by the sensor 521 through the processor 622. For example, the processor 622 may determine the position of the ultrasonic generator 400 based on the magnetic force detection result information by the two or more sensors 521a to 521f.

The step of determining the state of the ultrasonic generator may be determined by the processor 622 that the ultrasonic generator 400 is in one of the separated, detached, and stopped states from the transfer unit 610 in the case that the position of the ultrasonic generator 400 does not change while the transfer unit 610 is being driven S830. The processor 622 may determine at least one of the real-time position of the transducer 430, the movement stop phenomenon of the transducer 430, and whether the transducer 430 is detached from the transfer unit 610 based on the magnetic detection result information.

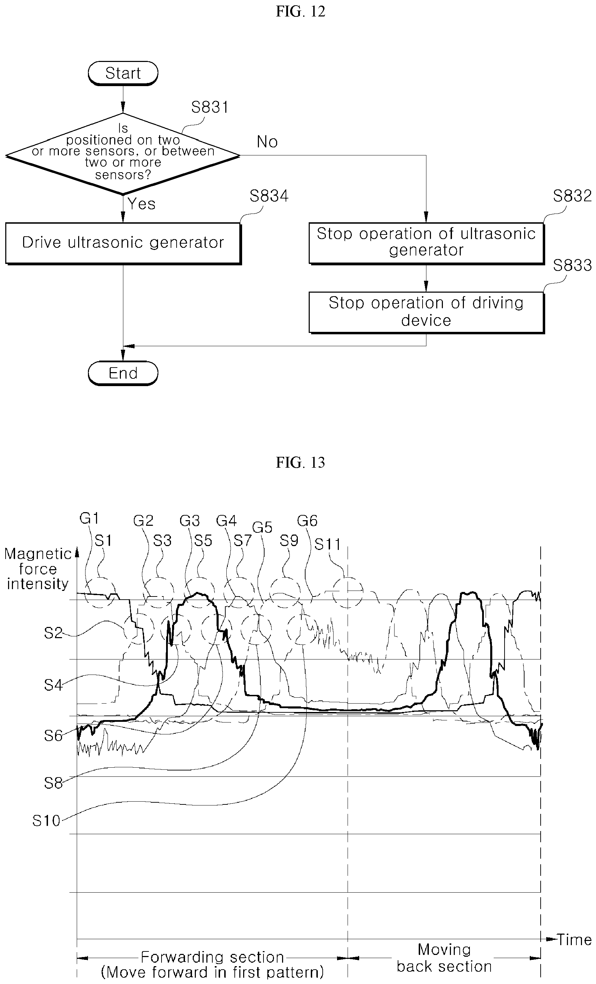

FIG. 12 is a flowchart illustrating an example of a method of irradiating ultrasonic energy by determining whether the ultrasonic generator and the driving device are driven based on the state determination of the ultrasonic generator of FIG. 8.

Referring to FIG. 12, the step of determining the state of the ultrasonic generator may determine whether the transducer 430 is changed to the position of the first sensor 521a to the sixth sensor 521f or is changed to the position between the first sensor 521a to the sixth sensor 521f through the processor 622 when the ultrasonic generator 400 moves in one of the first pattern as shown in FIG. 7 (a), the second pattern as shown in FIG. 7 (b), and the first pattern and the second pattern as shown in FIG. 7 (c) through the transfer unit 610 (step S831).

FIG. 13 is a diagram illustrating an example of a process of outputting magnetic detection result information through the processor when the ultrasonic generator of FIG. 7 moves forward in the first pattern.

Referring to FIG. 13, when the ultrasonic generator 400 moves forward in the first pattern and the transducer 430 changes to the position of the first sensor 521a to the sixth sensor 521f, the processor 622 may output magnetic detection result information for each linear graph G1 to G6 including multiple sensing points S1, S3, S5, S7, S9, and S11 having high magnetic strength. S1, S3, S5, S7, S9, and S11 represent when the transducer 430 sequentially changes to the position of the first sensor 521a to the sixth sensor 521f.

When the ultrasonic generator 400 moves forward in the first pattern, the processor 622 may output magnetic force detection result information for each linear graph G1 to G6 including the multiple sensing points S2, S4, S6, S8, and S10 where multiple magnetic forces intersect each other when the transducer 430 is changed to the position between the first sensor 521a to the sixth sensor 521f. S2, S4, S6, S8, and S10 represent when the transducer 430 is sequentially changed to the position between the first sensor 521a to the sixth sensor 521f.

When the ultrasonic generator 400 moves forward in the first pattern, the processor 622 may output magnetic force detection result information for each linear graph G1 to G6 including the multiple sensing points S1 to S11 in the order of S1 to S11.

Referring to FIG. 12, in the step of stopping the operation of the ultrasonic generator, in the case that the magnetic force detection result information is not based on the multiple sensing points S1, S3, S5, S7, S9, and S11 with high magnetic strength and the multiple sensing points S2, S4, S6, S8, and S10 where multiple magnetic forces intersect each other, the operation of the ultrasonic generator 400 may be stopped (step S832) so that the ultrasound of the ultrasonic generator 400 are not output by the processor 622. In the case that the magnetic detection result information is not the multiple sensing points S1, S3, S5, S7, S9, and S11 with high magnetic strength and the multiple sensing points S2, S4, S6, S8, and S10 where the multiple magnetic forces intersect each other, and there is no change in the position of the transducer 430 during the operation of the transfer unit 200, the processor 622 may determine that the transducer 430 is in the stopped state or is in one of the separated state or the detached state from the transfer unit 610. In the case that the magnetic force detection result information is not based on the multiple sensing points S1, S3, S5, S7, S9, and S11 with high magnetic force intensity and the multiple sensing points S2, S4, S6, S8, and S10 where the multiple magnetic forces intersect each other, the processor 622 may stop the operation of the transducer 430 to prevent thermal injury.

Referring to FIG. 12, the step of stopping the operation of the driving device may stop the operation of the driving device 200 through the processor 622 to prevent damage to the driving components and prevent additional accidents in the case that the magnetic force detection result information is not based on the multiple sensing points S1, S3, S5, S7, S9, and S11 with high magnetic force intensity and the multiple sensing points S2, S4, S6, S8, and S10 where the multiple magnetic forces intersect each other (step S833).

Referring to FIG. 12, the step of driving the ultrasonic generator may drive the ultrasonic generator 400 so that the ultrasonic generator 400 outputs ultrasound in the case that the magnetic force detection result information is based on the multiple sensing points S1, S3, S5, S7, S9, and S11 with high magnetic strength and the multiple sensing points S2, S4, S6, S8, and S10 where the multiple magnetic forces intersect each other through the processor 622 (step S834). The transducer 430 may irradiate ultrasound to the skin S on the movement path of the transfer unit 610. Since the transducer 430 irradiates ultrasound when moving forward in the first pattern and does not irradiate ultrasonic waves when moving back, it may be seen that the width of the magnetic field representing the linear graph G1 to G6 during the forward movement is wider than the width of the magnetic field representing the linear graph during the return movement.

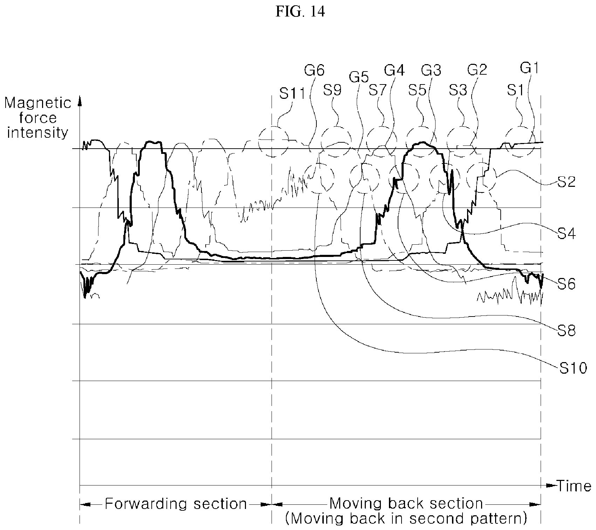

FIG. 14 is a diagram illustrating an example of a process of outputting magnetic detection result information through the processor when the ultrasonic generator of FIG. 7 moves back to the second pattern.

Referring to FIG. 14, when the ultrasonic generator 400 moves back to the second pattern, the processor 622 may output magnetic detection result information for each linear graph G6 to G1 including the multiple sensing points S11, S9, S7, S5, S3, and S1 having high magnetic strength when the transducer 430 changes to the position of the sixth sensor 521f to the first sensor 521a. S11, S9, S7, S5, S3, and S1 represent when the transducer 430 sequentially changes to the position of the sixth sensor 521f to the first sensor 521a.

When the ultrasonic generator 400 moves back to the second pattern, the processor 622 may output magnetic detection result information for each linear graph G6 to G1 including the multiple sensing points S10, S8, S6, S4, and S2 where multiple magnetic forces intersect each other when the transducer 430 is changed to a position between the sixth sensor 521f and the first sensor 521a. S10, S8, S6, S4, and S2 represent when the transducer 430 is sequentially changed to a position between the sixth sensor 521f and the first sensor 521a.

When the ultrasonic generator 400 moves back to the second pattern, the processor 622 may output magnetic detection result information for each linear graph G6 to G1 including the multiple sensing points S11 to S1 in the order of S11 to S1.

Referring to FIG. 12, in the step of stopping the operation of the ultrasonic generator, in the case that the magnetic force detection result information is not based on the multiple sensing points S11, S9, S7, S5, S3, and S1 with high magnetic strength and the multiple sensing points S10, S8, S6, S4, and S2 where the multiple magnetic forces intersect each other, the operation of the ultrasonic generator 400 may be stopped by the processor 622 (step S832) so that the ultrasound of the ultrasonic generator 400 are not output. In the case that the processor 622 determines that the magnetic detection result information is not based on the multiple sensing points S11, S9, S7, S5, S3, and S1 with high magnetic strength and the multiple sensing points S10, S8, S6, S4, and S2 where the multiple magnetic forces intersect each other, and there is no change in the position of the transducer 430 during the operation of the transfer unit 610, the processor 622 may determine that the transducer 430 is in one of the stopped state, the separated state, or the detached state from the transfer unit 610. In the case that the magnetic force detection result information is not based on the multiple sensing points S11, S9, S7, S5, S3, and S1 with high magnetic force intensity and the multiple sensing points S10, S8, S6, S4, and S2 where the multiple magnetic force intersects each other, the processor 622 may stop the operation of the transducer 430 to prevent thermal injury.

Referring to FIG. 12, the step of stopping the operation of the driving device may stop the operation of the driving device 200 through the processor 622 to prevent damage to the driving components and prevent an additional accident in the case that the magnetic force detection result information is not based on the multiple sensing points S11, S9, S7, S5, S3, and S1 with high magnetic force intensity and the multiple sensing points S10, S8, S6, S4, and S2 where the magnetic force intersects each other (step S833).

Referring to FIG. 12, the step of driving the ultrasonic generator may drive the ultrasonic generator 400 so that the ultrasound of the ultrasonic generator 400 is output in the case that the magnetic force detection result information is based on the multiple sensing points S11, S9, S7, S5, S3, and S1 with high magnetic force intensity and the multiple sensing points S10, S8, S6, S4, and S2 where the multiple magnetic forces intersect each other through the processor 622 (step S834). The transducer 430 may irradiate ultrasound to the skin S on the movement path of the transfer unit 610. Since the transducer 430 does not irradiate ultrasonic energy during forward movement and irradiates ultrasound during return movement in the second pattern, it may be seen that the width of the magnetic field representing the linear graph G6 to G1 during return movement is wider than the width of the magnetic field representing the linear graph during forward movement.

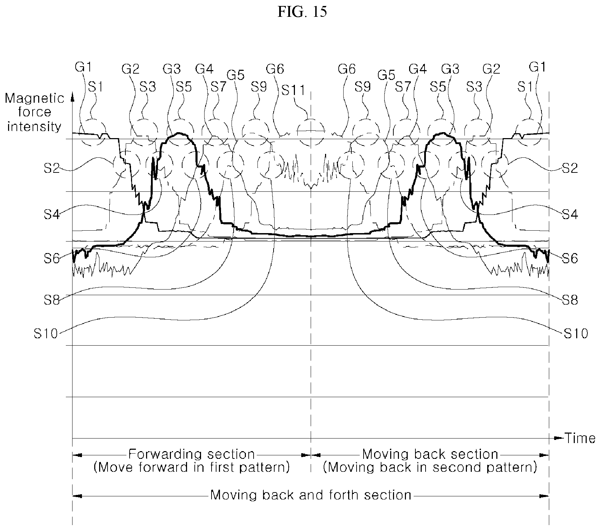

FIG. 15 is a diagram illustrating an example of a process for outputting magnetic detection result information through the processor when the ultrasonic generator of FIG. 7 moves back and forth in the first pattern and the second pattern.

Referring to FIG. 15, when the ultrasonic generator 400 moves forward in the first pattern and the transducer 430 changes to the position of the first sensor 521a to the sixth sensor 521f, the processor 622 may output magnetic detection result information for each linear graph G1 to G6 including the multiple sensing points S1, S3, S5, S7, S9, and S11 having high magnetic strength. S1, S3, S5, S7, S9, and S11 represent when the transducer 430 sequentially changes to the position of the first sensor 521a to the sixth sensor 521f.

When the ultrasonic generator 400 moves forward in the first pattern, the processor 622 may output magnetic force detection result information for each linear graph G1 to G6 including the multiple sensing points S2, S4, S6, S8, and S10 where the multiple magnetic forces intersect each other when the transducer 430 is changed to a position between the first sensor 521a to the sixth sensor 521f. S2, S4, S6, S8, S10 represent when the transducer 430 is sequentially changed to a position between the first sensor 521a to the sixth sensor 521f.

When the ultrasonic generator 400 moves forward in the first pattern, the processor 622 may output magnetic force detection result information for each linear graph G1 to G6 including multiple sensing points S1 to S11 in the order of S1 to S11.

When the ultrasonic generator 400 moves back to the second pattern, the processor 622 may output magnetic detection result information for each linear graph G6 to G1 including the multiple sensing points S11, S9, S7, S5, S3, and S1 having high magnetic strength when the transducer 430 is changed to the position of the sixth sensor 521f to the first sensor 521a. S11, S9, S7, S5, S3, and S1 represent when the transducer 430 is sequentially changed to the position of the sixth sensor 521f to the first sensor 521a.

When the ultrasonic generator 400 moves back to the second pattern, the processor 622 may output magnetic detection result information for each linear graph G6 to G1 including the multiple sensing points S10, S8, S6, S4, and S2 where multiple magnetic forces intersect each other when the transducer 430 is changed to a position between the sixth sensor 521f and the first sensor 521a. S10, S8, S6, S4, and S2 represent when the transducer 430 is sequentially changed to the position between the sixth sensor 521f and the first sensor 521a.

When the ultrasonic generator 400 moves back to the second pattern, the processor 622 may output magnetic detection result information for each linear graph G6 to G1 including the multiple sensing points S11 to S1 in the order of S11 to S1.

Referring to FIG. 12, in the step of stopping the operation of the ultrasonic generator, in the case that the magnetic force detection result information is not based on the multiple sensing points S1, S3, S5, S7, S9, and S11 with high magnetic strength and the multiple sensing points S2, S4, S6, S8, and S10 where the multiple magnetic forces intersect each other, the operation of the ultrasonic generator 400 may be stopped by the processor 622 (step S832) so that the ultrasound of the ultrasonic generator 400 are not output. The processor 622 may determine that the magnetic detection result information is based on the multiple sensing points S1, S3, S5, S7, S9, and S11 having high magnetic strength and the multiple sensing points S2, S4, S6, S8, and S10 where the multiple magnetic forces intersect each other, and in the case that there is no change in the position of the transducer 430 during the operation of the transfer unit 610, the transducer 430 is in the stopped state, or is in one of the separated state and the stopped state from the transfer unit 610. The processor 622 may prevent thermal injury by stopping the operation of the transducer 430 in the case that the magnetic force detection result information is not based on the multiple sensing points S1, S3, S5, S7, S9, and S11 with high magnetic force intensity and the multiple sensing points S2, S4, S6, S8, and S10 where the magnetic forces intersect each other.

In addition, the step of stopping the operation of the ultrasonic generator may stop the operation of the ultrasonic generator 400 so that the ultrasound of the ultrasonic generator 400 are not output in the case that the magnetic force detection result information is not based on the multiple sensing points S11, S9, S7, S5, S3, and S1 with high magnetic force intensity and the multiple sensing points S10, S8, S6, S4, and S2 where the magnetic forces intersect each other (step S832). In the case that the magnetic detection result information is not based on the multiple sensing points S11, S9, S7, S5, S3, and S1 with high magnetic strength and the multiple sensing points S10, S8, S6, S4, and S2 where the multiple magnetic forces intersect each other, and there is no change in the position of the transducer 430 during the operation of the transfer unit 610, the processor 622 may determine that the movement of the transducer 430 has stopped or that the transducer 430 has been separated from the transfer unit 610. In the case that the magnetic detection result information is not based on the multiple sensing points S11, S9, S7, S5, S3, and S1 with high magnetic strength and the multiple sensing points S10, S8, S6, S4, and S2 where the magnetic forces intersect each other, the processor 622 may stop the operation of the transducer 430 to prevent thermal injury.

Referring to FIG. 12, the step of stopping driving of the driving device may prevent damage to the driving components and prevent an additional accident by stopping the driving of the driving device 200 in the case that the magnetic force detection result information through the processor 622 is not based on the multiple sensing points S1, S3, S5, S7, S9, and S11 with high magnetic force intensity and the multiple sensing points S2, S4, S6, S8, and S10 where the multiple magnetic forces intersect each other (step S833).

In addition, the step of stopping driving of the driving device may prevent damage to the driving components and prevent an additional accident by stopping the driving of the driving device 200 in the case that the magnetic force detection result information through the processor 622 is not based on the multiple sensing points S11, S9, S7, S5, S3, and S1 with high magnetic force intensity and the multiple sensing points S10, S8, S6, S4, and S2 where the multiple magnetic forces intersect each other (step S833).

Referring to FIG. 12, the ultrasonic generator driving step may drive the ultrasonic generator 400 so that ultrasonic waves of the ultrasonic generator 400 are output in the case that the magnetic force detection result information is based on the multiple sensing points S1, S3, S5, S7, S9, and S11 with high magnetic strength and the multiple sensing points S2, S4, S6, S8, and S10 where the multiple magnetic forces intersect each other (step S834) through the processor 622. The transducer 430 may irradiate ultrasound to the skin S on the movement path of the transfer unit 610.

In addition, the step of driving the ultrasonic generator may drive the ultrasonic generator 400 so that ultrasonic energy of the ultrasonic generator 400 is output in the case that the magnetic force detection result information through the processor 622 is based on the multiple sensing points S11, S9, S7, S5, S3, and S1 having high magnetic strength and the multiple sensing points S10, S8, S6, S4, and S2 where the multiple magnetic forces intersect each other (step S834). The transducer 430 may irradiate ultrasound to the skin S on the movement path of the transfer unit 610. Since the transducer 430 irradiates ultrasound when moving forward in the first pattern and irradiates ultrasonic waves when moving back in the second pattern, it may be seen that the width of the magnetic field representing the linear graph G1 to G6 during the forward movement is the same as the width of the magnetic field representing the linear graph G6 to G1 during the return movement. The processor 622 may notify the return state through a notification unit when changing from forward movement to return movement. For example, the notification unit may include a display module that visually notifies, and a speaker that auditorily notifies.

The step of driving the ultrasonic generator according to the present disclosure may further control so that ultrasound of the same condition or ultrasound of different conditions are output through the processor 622 (step S834). At this time, the condition may include at least one of the time for which ultrasonic energy irradiation is maintained or the intensity of ultrasonic energy.

The step of driving the ultrasonic generator may prevent the occurrence of a risk due to burns in advance by controlling high heat and high ultrasonic energy.

That is, as illustrated in FIG. 7, the step of driving the ultrasonic generator may further control so that the first time t1 for which ultrasonic energy irradiation is maintained when the ultrasonic generator 400 moves in the first pattern and the second time t2 for which ultrasonic energy irradiation is maintained when it moves in the second pattern are output differently through the processor 622.

For example, the first time t1 during which the irradiation of ultrasonic energy is maintained during the forward movement in the first pattern may be earlier or later than the second time t2 during which the irradiation of ultrasonic energy is maintained during the return movement in the second pattern.

In addition, as illustrated in FIG. 7, the step of driving the ultrasonic generator may further control the ultrasonic generator 400 to output the first intensity E1 of ultrasonic energy during the forward movement in the first pattern and the second intensity E2 of ultrasonic energy during the return movement in the second pattern differently through the processor 622.

For example, the first intensity E1 of ultrasonic energy during the forward movement in the first pattern may be greater or less than the second intensity E2 of ultrasonic energy during the return movement in the second pattern. At least one component may be added or deleted in accordance with the performance of the components illustrated in FIG. 5. In addition, it will be readily understood by those skilled in the art that the mutual positions of the components may be changed in accordance with the performance or structure of the system.

Although FIGS. 8 and 12 describe the execution of multiple steps sequentially, this is merely an exemplary description of the technical idea of the present embodiment, and those skilled in the art to which the present embodiment belongs may modify and change the order described in FIGS. 8 and 12 without departing from the essential features of the present embodiment, or may execute one or more of the multiple steps in parallel, thereby applying various modifications and variations. Therefore, FIGS. 8 and 12 are not limited to a sequential order.

As described above, the disclosed embodiments have been described with reference to the attached drawings. Those skilled in the art to which the present disclosure belongs will understand that the present disclosure may be implemented in forms other than the disclosed embodiments without changing the technical idea or essential features of the present disclosure. The disclosed embodiments are exemplary and should not be construed as limiting.

## Claims

1. An ultrasonic generation device comprising:
an ultrasonic generator;
a transfer unit coupled to the ultrasonic generator and configured to move the ultrasonic generator in a preset pattern;
a sensor configured to detect a movement of the ultrasonic generator; and
a control unit configured to:
determine a position of the ultrasonic generator based on detection result information by the sensor, and
based on the position of the ultrasonic generator not changing while the transfer unit is being driven, determine that the ultrasonic generator is in one of a state of being separated from the transfer unit, a state of being detached, and a state of being stopped.

2. The device of claim 1, wherein two or more of the sensors are installed at an equal interval along a moving path of the ultrasonic generator.

3. The device of claim 1, wherein the control unit is configured to determine the position of the ultrasonic generator based on magnetic force detection result information from the sensor.

4. The device of claim 3,
wherein the preset pattern includes at least one of a first pattern in which the ultrasonic generator moves from a first point to a second point, or a second pattern in which the ultrasonic generator moves from the second point to the first point, and
wherein the magnetic force detection result information includes multiple sensing points having a high intensity of the magnetic force among detected multiple magnetic forces, and multiple sensing points where the multiple magnetic forces intersect each other.

5. The device of claim 4, wherein the control unit is further configured to:
when the ultrasonic generator moves in one of the first pattern and the second pattern,
stop an operation of the ultrasonic generator so that an ultrasound from the ultrasonic generator is not output based on the magnetic force detection result information being not based on the multiple sensing points with the high intensity of the magnetic force and the multiple sensing points where the multiple magnetic forces intersect each other.

6. The device of claim 1, further comprising a cartridge housing provided to accommodate the ultrasonic generator and is coupled to one side of a handpiece, and
wherein the handpiece includes an engaging projection formed on an inner surface and a main drive shaft having magnetism configured to couple with the cartridge housing,
wherein the cartridge housing includes an insertion groove formed on an outer surface and a magnetic body provided to face the main drive shaft, and
wherein the cartridge housing, when coupled with the handpiece, the insertion groove is engaged by the engaging projection and is magnetically coupled with the main drive shaft having magnetism by the magnetic body.

7. A method for determining and controlling a state of an ultrasonic generation device, the method comprising:
moving an ultrasonic generator in a preset pattern;
detecting a movement of the ultrasonic generator; and
determining a position of the ultrasonic generator based on detection result information by a sensor; and
based on the position of the ultrasonic generator not changing while the transfer unit is being driven, determining that the ultrasonic generator is in one of a state of being separated from the transfer unit, a state of being detached, and a state of being stopped.

8. The method of claim 7, wherein the position of the ultrasonic generator is determined based on magnetic force detection result information from the sensor.

9. The method of claim 8,
wherein the preset pattern includes at least one of a first pattern in which the ultrasonic generator moves from a first point to a second point, or a second pattern in which the ultrasonic generator moves from the second point to the first point, and
wherein the magnetic force detection result information includes multiple sensing points having a high intensity of the magnetic force among detected multiple magnetic forces, and multiple sensing points where the multiple magnetic forces intersect each other.

10. The method of claim 9, further comprising:
when the ultrasonic generator moves in one of the first pattern and the second pattern,
stopping an operation of the ultrasonic generator so that an ultrasound from the ultrasonic generator is not output based on the magnetic force detection result information being not based on the multiple sensing points with the high intensity of the magnetic force and the multiple sensing points where the multiple magnetic forces intersect each other.
